# EUROPEAN PATENT SPECIFICATION

(11) **EP 4 444 687 B1**
(45) Date of publication and mention of the grant of the patent: **02.09.2026**
(21) Application number: 23714294.8
(22) Date of filing: 25.02.2023
(51) Int. Cl.: C07C 29/141, C07C 47/02, C07C 47/21, C07C 45/74, C07C 45/50, C07C 31/125, C07C 31/12, B01D 53/62, B01D 53/78, B01D 53/86, C01B 3/56, B01D 53/047, C01B 3/00, B01D 53/82, C01B 3/38, C01B 3/52

(54) **AN INTEGRATED PROCESS FOR PRODUCING OXO ALCOHOLS FROM NATURAL GAS**
INTEGRIERTES VERFAHREN ZUR HERSTELLUNG VON OXOALKOHOLEN AUS ERDGAS
PROCÉDÉ INTÉGRÉ DE PRODUCTION D'OXO-ALCOOLS À PARTIR DE GAZ NATUREL

(30) Priority: 27.02.2022 IR 14003009301
(43) Date of publication of application: 16.10.2024
(73) Proprietor: PETROSAKHT CHEHELSOTON ENGINEERING TECHNICAL COMPANY, Esfehan (IR)
(72) Inventor: GHARAVI, Mohammadreza, Tehran (IR); ZEREHSAZ, Mohammadhadi, Mashhad (IR); GHARAVI, Alaodin, Arak (IR)
(74) Representative: Fuchs Patentanwälte Partnerschaft mbB
(86) International application number: PCT/IB2023/051763
(87) International publication number: WO 2023/161884

(56) References cited:
- EP-A1- 2 261 172
- WO-A1-2019/197831

## Description

### Technical Field

This invention relates to an integrated process for producing oxo alcohols including 2-ethylhexanol, n-butanol and iso-butanol from natural gas. Oxo alcohols are produced through reacting synthesis gas with an olefin to obtain an aldehyde using hydroformylation reaction and then hydrogenation of the aldehyde to obtain the alcohol.

Synthesis gas or syngas is a mixture of carbon monoxide (CO) and hydrogen in various ratios. Steam reforming of natural gas is a route of syngas production. The molar ratio of CO:H2 is 1:3.

CH₄ + H₂O → CO + 3 H₂

However, syngas may be produced through reacting natural gas with carbon dioxide. The molar ratio of CO:H2 is 1:1 [1].

CH₄ + CO2 → 2 CO + 2 H₂

Since natural gas is used as feed in the present process, this process integrates different stages such as syngas production, CO2 removal, hydrogen purification, production of aldehydes, aldolization, hydrogenation, and finally continuous catalyst regeneration.

### Background Art

According to CEH report published by S&P Global Inc. , the oxo process or hydroformylation of olefins with synthesis gas is principal route to C₃-C₁₅ aldehydes, which are converted into alcohols, acids, or other derivatives. By far the most important oxo chemical is n-butyraldehyde, followed by C₆-C₁₃ aldehydes for plasticizer alcohols, iso-butyraldehyde, propionaldehyde, valeraldehyde, and C₁₂-C₁₈ aldehydes for detergent alcohols [2]. However, research activities are continually performed in order to develop the oxo technology in the world.

U.S. Patent Publication No. 2021/0179425 discloses how to produce pure hydrogen using a membrane system with a PSA (Pressure Swing Adsorption) unit. To produce hydrogen, first the syngas is de-acidified and enters a membrane, and then the remaining flow enters the PSA to produce hydrogen.

International Patent Publication No. WO 2019/064107 discloses use of bisulfite compound for reacting with aldehydes and separate it from the oxo alcohol product to increase purity of the produced alcohols.

International Patent Publication No. WO 2016/103277 discloses synthesis of C3 - C4 oxo alcohols from corresponding aldehyde with greater selectivity in a single pot using recyclable multifunctional catalyst. The modified process selectively prepare C3 - C4 oxo alcohols in single pot which results in simple process, reduction in reaction time, reaction steps and overall cost of manufacturing.

International Patent Publication No. WO 2008/147129 discloses a device for better phase contact in a hydroformylation reaction of olefins. This device increases the contact between liquid and gas phase and increases efficiency of hydroformylation of olefins. Document WO 2019/197831 discloses a process for the production of n-butanol, iso-butanol and 2-alkyl alkanol starting from n-butyraldehyde, iso-butyraldehyde and 2-alkyl alkenal.

### Summary of Invention

The invention discloses an integrated process for producing oxo alcohols, including 2-ethylhexanol, n-butanol and iso-butanol from natural gas. In addition to the integration, the process has the unique features such as reusing carbon dioxide and unreacted propylene, continuous catalyst regeneration, using the separated heavy alcohols as fuel, improved system of alcohol separation and improved aldolization system with a less loop reactor.

### Technical Problem

Production of oxo alcohols including 2-ethylhexanol, n-butanol and iso-butanol through hydroformylation reaction requires a significant amount of pure hydrogen for hydrogenation of oxo aldehydes and also syngas for hydroformylation of propylene as the olefin. In addition, the process requires steam of water for operating heat exchangers in the alcohol separation unit. Obviously, supply of these items from the outside of the plant can be problematic in terms of their purchase price and the required technical specifications.

In addition to the above, reducing fixed investment and production cost and minimizing the adverse environmental effects of production have always been the favorites of petrochemical unit owners.

### Solution to Problem

This invention discloses an integrated process for producing oxo alcohols including 2-ethylhexanol, n-butanol and iso-butanol from natural gas. In addition to the integration, the process has a number of exclusive features as below:
1. Recycling carbon dioxide produced in the reforming unit;
2. Recycling propylene unreacted in the hydroformylation reaction;
3. Using the separated heavy alcohols as fuel in furnace of the reforming unit;
4. Continuous regeneration of hydroformylation catalyst;
5. Removing a loop reactor in the butyraldehyde aldolization unit; and
6. Improved separation operation of the alcohols.

### Advantageous Effects of Invention

Due to above unique features, the process is able to provide the required items i.e. pure hydrogen, synthesis gas and steam of water without dependence on the outside of the plant, as well as reducing common concerns in petrochemical units, including high fixed investment, high production cost and environmental issues as much as possible. Advantages of the invented process include:
1. Reducing natural gas feed consumption and adverse environmental effects due to recycling carbon dioxide produced in the reforming unit and its reuse in syngas production;
2. Decreasing production cost due to recycling of the unreacted propylene in the oxo reactor;
3. Increasing efficiency of the process due to reusing of the unreacted propylene in hydroformylation reaction;
4. Reducing fixed investment and energy consumption due to elimination of a loop reactor in the aldolization unit;
5. Reducing fuel consumption due to using of the separated heavier alcohols in furnace of the reforming unit; and
6. Due to continuous operations of the catalyst regeneration and the oxo alcohols separation, the invented process operates as continuous with less annual waste.

### Brief Description of Drawings

### Fig.1

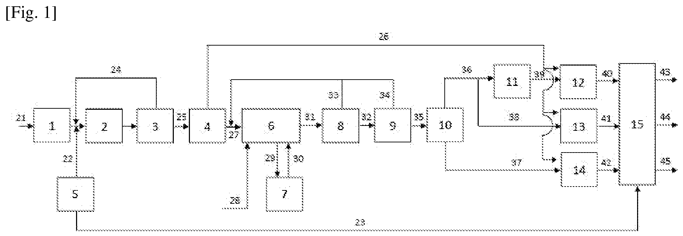

[Fig.1] shows block flow diagram of the process for producing the oxo alcohols i.e. 2-ethylhexanol, n-butanol and iso-butanol from natural gas. The units in the process, according to their number in the diagram, are as follows:

| | | | |
|---|---|---|---|
| 1 | Desulfurization | 9 | Stabilization |
| 2 | Reforming | 10 | Separation of isomers |
| 3 | CO2 removal | 11 | Aldolization |
| 4 | Pressure Swing Adsorption (PSA) | 12 | Hydrogenation |
| 5 | Steam production | 13 | Hydrogenation |
| 6 | Hydroformylation (oxo reactor) | 14 | Hydrogenation |
| 7 | Catalyst regeneration | 15 | Separation of oxo alcohols |
| 8 | Stripping | | |

The flow lines are as below:

| | | | |
|---|---|---|---|
| 21 | Natural gas | 34 | Recycled gases |
| 22 | Steam of water | 35 | Mixture of pure aldehydes |
| 23 | Steam of water | 36 | n-butyraldehyde |
| 24 | CO2 gas | 37 | Iso-butyraldehyde |
| 25 | CO2 free syngas | 38 | n-butyraldehyde |
| 26 | Pure hydrogen | 39 | 2-ethyl-3-propylacrolein |
| 27 | Synthesis gas | 40 | Impure 2-ethylhexanol |
| 28 | Propylene | 41 | Impure n-butanol |
| 29 | Inactive catalyst | 42 | Impure iso-butanol |
| 30 | Regenerated catalyst | 43 | Pure 2-ethylhexanol |
| 31 | Mixture of impure aldehydes | 44 | Pure n-butanol |
| 32 | Mixture of impure aldehydes | 45 | Pure iso-butanol |
| 33 | Recycled gases | | |

### Description of Embodiments

The invention discloses an integrated process for producing oxo alcohols including 2-ethylhexanol, n-butanol and iso-butanol from natural gas. The integration causes the required items including pure hydrogen, synthesis gas and steam of water to be supplied without dependence on the outside. On the other hand, the unique features of the process reduce fixed investment, high production cost and environmental issues as much as possible.

In this process, natural gas is converted into the oxo alcohols i.e. 2- ethylhexanol (43), n-butanol (44) and isobutanol (45) after passing via different process steps.

In first step, natural gas (21) enters the desulfurization unit (1) and then is purified in the presence of an alumina-based molybdenum/cobalt oxide catalyst at temperature range of 360 to 380 °C. After the purification, it is transferred to the reforming unit (2) with maximum sulfur content of 1 ppm.

After that, sulfur free natural gas enters the reforming unit and then is converted into syngas in the presence of the steam and an alumina-based nickel catalyst at temperature range of 700 to 900 °C and pressure of 12 to 16 bar. Along with natural gas, carbon dioxide gas (24), which is recycled from the CO2 removal unit (3), participates in producing the syngas. The pressure and temperature of the steam (22) entering the reforming unit, which is supplied by the steam production unit (5), are in the range of 15 to 25 bar and 200 to 250 °C, respectively.

Next, the syngas enters the CO2 removal unit (3). In this step, carbon dioxide produced during the reforming process is absorbed and returned to the reforming unit (2) through a return flow line (24).

Under purification operation in the PSA unit (4) in the presence of the syngas, hydrogen gas is produced with minimum purity of 99.5%. The pure hydrogen gas is transferred to the hydrogenation units (12), (13) and (14) via flow line (26).

The syngas passes through the PSA unit and enters the oxo reactor (6) via flow line (27). Before entering the reactor, the ratio of hydrogen to carbon monoxide in the syngas is between 1.01 and 1.05.

In the oxo reactor, n-butyraldehyde and iso-butyraldehyde are produced under hydroformylation reaction of the propylene with the syngas. The propylene is supplied from the outside (28) and from the unreacted propylene recycled from the stripping unit (8) and the stabilization unit (9) via flow lines (33) and (34), respectively.

Due to the unique design of the catalyst regeneration system (7), in which continuous replacement of up to 15 % vol. of the inactive catalyst (29) with the regenerated catalyst (30) is possible, the hydroformylation is carried out as a continuous reaction in the reactor. In the catalyst regeneration system (7), the inactive catalyst is replaced with the regenerated catalyst when temperature of the reactor exceeds 110 °C.

The product of the reaction is a mixture of the butyraldehydes along with dissolved gases including carbon monoxide, hydrogen, methane, propane and unreacted propylene. By passing the product (31) through the stripping unit (8) and then the stabilization unit (9), the gases separate from the butyraldehydes and then return to the reactor via flow lines (33) and (34).

Next, the mixture (35) enters the separation unit of the butyraldehyde isomers (10). In this step, n-butyraldehyde and iso-butyraldehyde separate from each other.

After that, n-butyraldehyde enters the aldolization system (11) via flow line (36). The aldolization system has been equipped with a loop reactor and a loop heat exchanger. In this system, n-butyraldehyde is converted into 2-ethyl-3-propyl acrolein (EPA) with minimum conversion of 95%. Through flow line (39), EPA enters the hydrogenation unit (12) to be converted into 2-ethylhexanol in the presence of the pure hydrogen supplied from the PSA unit (4). The conversion rate of this reaction is at least 94%.

In addition, n-butyraldehyde (38) can be converted into n-butanol in the presence of the pure hydrogen supplied from PSA unit (4) in the hydrogenation unit (13). The conversion rate of this reaction is 99%.

Through flow line (37), iso-butyraldehyde is converted into iso-butanol in the presence of the pure hydrogen supplied from PSA unit (4) in the hydrogenation unit (14). The conversion rate of this reaction is 99%.

Finally, each of the alcohols i.e. 2-ethylhexanol (40), n-butanol (41) and iso-butanol (42) is continuously purified in the separation unit of oxo alcohols (15) and then transferred to the storage tanks via flow lines (43), (44) and (45), respectively.

It is important to note that the unique design of the separation unit of oxo alcohols causes the unit to operate as continuous. The steam production unit (5) supplies the required the steam for heat exchangers of the separation unit through flow line (23). The separated heavier alcohols are consumed as fuel in furnace of the reforming unit.

### Examples

The amount of 3500 cubic meters per hour of natural gas as feed and the amount of 1300 cubic meters per hour as fuel for the reforming unit enter the oxo alcohol plant. Table (1) presents specification of the feed.

Natural gas enters the reforming unit after desulfurization at temperature of 380 °C and pressure of 18 bar with a sulfur content of 1 ppm. Using furnace of the reforming unit, the amount of 14 tons of the steam is produced in the steam production unit, which is used for heat exchangers of the oxo alcohols separation unit. The syngas produced in the reforming unit enters the CO2 removal unit to separate the carbon dioxide. The separated CO2 return to the reforming unit.

After removing the carbon dioxide, the syngas enters the PSA unit to produce hydrogen with a purity of 99.5% to supply the required hydrogen for the hydrogenation units. The produced syngas has a molar ratio of hydrogen to carbon monoxide of 1.03.

This gas enters the oxo reactor together with the propylene gas. After reducing the activity of the oxo reactor catalyst, using an innovative design, 12% of volume of the inactive catalyst is replaced with the regenerated catalyst. For this reason, the reactor operates continuously and efficiently. The reaction product is the mixture of butyraldehydes, which contains the dissolved gases. These gases successively separate in the stripping and stabilization units. The purity of n-butyraldehyde and iso-butyraldehyde at this stage is 99.5% and 93%, respectively.

8600 kg per hour of n-butyraldehyde is converted into 2-ethylhexanol under the aldolization reaction and then the hydrogenation. The purity and amount of the alcohol are 99.8% and 8100 kg per hour, respectively. In addition, n-butyraldehyde can be directly hydrogenated and converted into n-butanol. The purity and amount of the alcohol are 99.8% and 9600 kg per hour, respectively.

Iso-butyraldehyde with the amount of 950 kg per hour is first stored and then converted into iso-butanol under the hydrogenation. The purity and amount of the alcohol are 99.8% and 9600 kg per hour, respectively.Table 1 indicates specification of the natural gas feed and the products.

**[Table 1]**

| **Stream** | **Purity (% mole)** | **Amount** | **Unit** |
|---|---|---|---|
| Natural gas | Methane (87%) | 4800 | m³/hr |
| | Ethane (4%) | | |
| | N2 (6%) | | |
| | C3+ (3%) | | |
| Iso-butyraldehyde | 93% | 950 | kg/hr |
| n-butyraldehyde | 99.5% | 8600 | kg/hr |
| 2-ethylhexanol | 99.8% | 8100 | kg/hr |
| n-buthanol | 99.8% | 9600 | kg/hr |
| Iso-buthanol | 99.8% | 9600 | kg/hr |

### Industrial Applicability

Considering importance of the integration in petrochemical processes through converting gas feed into valuable products in terms of economic aspects and reduction of dependence on the outside of the plant, this invention is applicable for production of oxo alcohols including: 2-ethylhexanol, n-butanol and iso-butanol from natural gas.

### Reference Signs List

[1] A. A. Tountas, X. Peng, A. V. Tavasoli, P. N. Duchesne, T. L. Dingle, Y. Dong, L. Hurtado, A. Mohan, W. Sun, U. Ulmer, L. Wang, T. E. Wood, C. T. Maravelias, M. M. Sain, and G. A. Ozin, *"Towards Solar Methanol: Past, Present, and Future",* ADVANCED SCIENCE, 6 (8), 2019.
[2] CEH Report, *"Oxo Chemicals",* S&P Global Inc., 2021.

### Reference to Deposited Biological Material

### Sequence Listing Free Text

### Citation List

Citation List follows:

### Patent Literature

PTL 1: U.S. Patent Publication 2021/0179425
PTL 2: International Patent Publication WO 2019/064107
PTL 3: International Patent Publication WO 2016/103277
PTL 4: International Patent Publication WO 2008/147129

### Non Patent Literature

NPL1:

## Claims

1. An integrated process for producing oxo alcohols from natural gas, including the following steps:
a) converting the desulfurized natural gas along with carbon dioxide gas recycled from the CO2 removal unit into the syngas in the reforming unit;
b) separating the CO2 gas from the syngas and returning it to the reforming unit;
c) purifying the syngas in order to produce the pure hydrogen which is used for hydrogenation of the oxo aldehydes;
d) producing n-butyraldehyde and iso-butyraldehyde through continuous hydroformylation reaction of the propylene with the syngas in the oxo reactor;
e) separating the dissolved gases from the mixture of butyraldehydes and returning them to the oxo reactor;
f) separating iso-butyraldehyde and n-butyraldehyde from each other;
g) producing 2-ethyl-3-propylacrolein through aldolization of n-butyraldehyde;
h) producing 2-ethylhexanol through hydrogenation of 2-ethyl-3-propylacrolein;
i) producing n-butanol and iso-butanol through hydrogenation of n- butyraldehyde and iso-butyraldehyde; and
j) continuously purifying the produced oxo alcohols including 2-ethylhexanol, n-butanol and iso-butanol.

2. The process of claim 1, wherein natural gas is purified in the desulfurization unit in the presence of an alumina-based molybdenum/cobalt oxide catalyst at temperature range of 360 to 380 °C up to maximum sulfur content of 1 ppm, and then the purified gas, along with carbon dioxide recycled from the CO2 removal unit, is converted into syngas in the presence of the steam and an alumina-based nickel catalyst at temperature range of 700 to 900 °C and pressure of 12 to 16 bar in the reforming unit.

3. The process of claim 2, wherein the steam is supplied by the steam production unit with a pressure of 15 to 25 bar and a temperature of 200 to 250 °C.

4. The process of claim 1, wherein the carbon dioxide produced in the reforming process is absorbed in the CO2 removal unit and then returned to the reforming unit.

5. The process of claim 1, wherein the pure hydrogen is produced with minimum purity of 99.5% after passing the CO2 free syngas through the pressure swing adsorption unit in order to use in hydrogenation of the oxo aldehydes.

6. The process of claim 1, wherein molar ratio of hydrogen to carbon monoxide in the syngas before entering to the oxo reactor is between 1.01 and 1.05.

7. The process of claim 1, wherein the propylene along with the syngas is converted into n-butyraldehyde and iso-butyraldehyde under continuous hydroformylation reaction in the oxo reactor.

8. The process of claim 7, wherein the propylene is supplied from the outside and from the unreacted propylene recycled from the stripping and stabilization units.

9. The process of claim 1, wherein due to continuous replacement of up to 15 % vol. of the inactive catalyst with the regenerated catalyst, the hydroformylation is carried out as a continuous reaction.

10. The process of claim 9, wherein the catalyst regeneration system replaces the inactive catalyst with the regenerated catalyst when temperature of the oxo reactor exceeds 110 °C.

11. The process of claim 1, wherein the gases dissolved in the mixture of butyraldehydes including carbon monoxide, hydrogen, methane, propane and unreacted propylene successively separate in the stripping and stabilizing units and then return to the oxo reactor.

12. The process of claim 1, wherein the mixture of aldehydes enters the separation unit of the isomers in order to separate iso-butyraldehyde and n-butyraldehyde from each other.

13. The process of claim 1, wherein n-butyraldehyde is converted into 2-ethyl-3-propylacrolein with minimum conversion of 95% after passing through the aldolization system equipped with a loop reactor and a loop heat exchanger.

14. The process of claim 1, wherein 2-ethyl-3-propylacrolein is converted into 2-ethylhexanol with minimum conversion of 94% in the hydrogenation unit in which the pure hydrogen supplied from the pressure swing adsorption unit.

15. The process of claim 1, wherein n-butyraldehyde and iso-butyraldehyde are separately converted into n-butanol and iso-butanol, respectively, with conversion rate of 99% through hydrogenation reaction in which the pure hydrogen supplied from the pressure swing adsorption unit.

16. The process of claim 1, wherein each of the produced oxo alcohols including 2-ethylhexanol, n-butanol and iso-butanol is continuously purified in the separation unit.

17. The process of claim 16, wherein steam required for heat exchangers of the separation unit is supplied by the steam production unit.

18. The process of claim 16, wherein the heavier alcohols separated from the separation unit are consumed as fuel in furnace of the reforming unit.

## Patentansprüche

1. Integriertes Verfahren zur Herstellung von Oxoalkoholen aus Erdgas, einschließend die folgenden Schritte:
a) Umwandeln des entschwefelten Erdgases zusammen mit Kohlendioxidgas recycelt von der CO2-Entfernungseinheit in das Synthesegas in der Reformierungseinheit;
b) Abtrennen des CO2-Gases von dem Synthesegas und Rückführen in die Reformierungseinheit;
c) Reinigen des Synthesegases, um den reinen Wasserstoff herzustellen, der zur Hydrierung der Oxoaldehyde verwendet wird;
d) Herstellen von n-Butyraldehyd und iso-Butyraldehyd durch kontinuierliche Hydroformylierungsreaktion von dem Propylen mit dem Synthesegas in dem Oxoreaktor;
e) Abtrennen der gelösten Gase von dem Gemisch von Butyraldehyden und Rückführen derselben in den Oxoreaktor;
f) Trennen von iso-Butyraldehyd und n-Butyraldehyd voneinander;
g) Herstellen von 2-Ethyl-3-propylacrolein durch Aldolisierung von n-Butyraldehyd;
h) Herstellen von 2-Ethylhexanol durch Hydrierung von 2-Ethyl-3-propylacrolein;
i) Herstellen von n-Butanol und iso-Butanol durch Hydrierung von n-Butyraldehyd und iso-Butyraldehyd; und
j) kontinuierlich Reinigen der hergestellten Oxoalkohole einschließend 2-Ethylhexanol, n-Butanol und iso-Butanol.

2. Verfahren nach Anspruch 1, wobei Erdgas in der Entschwefelungseinheit in der Gegenwart eines Molybdän-/Kobaltoxid-Katalysators auf Aluminiumoxid-Basis in einem Temperaturbereich von 360 bis 380 °C bis zu einem maximalen Schwefelgehalt von 1 ppm gereinigt wird, und dann das gereinigte Gas, zusammen mit Kohlendioxidgas recycelt von der CO2-Entfernungseinheit, in Synthesegas in der Gegenwart des Dampfes und eines Nickel-Katalysators auf Aluminiumoxid-Basis in einem Temperaturbereich von 700 bis 900 °C und unter einem Druck von 12 bis 16 bar in der Reformierungseinheit umgewandelt wird.

3. Verfahren nach Anspruch 2, wobei der Dampf durch die Dampfherstellungseinheit mit einem Druck von 15 bis 25 bar und einer Temperatur von 200 bis 250 °C zugeführt wird.

4. Verfahren nach Anspruch 1, wobei das in dem Reformierungsverfahren hergestellte Kohlendioxid in der CO2-Entfernungseinheit absorbiert wird und dann in die Reformierungseinheit rückgeführt wird.

5. Verfahren nach Anspruch 1, wobei der reine Wasserstoff mit minimaler Reinheit von 99,5% hergestellt wird, nach Passieren des CO2-freien Synthesegases durch die Druckwechseladsorptionsanlage, um in der Hydrierung der Oxoaldehyde verwendet zu werden.

6. Verfahren nach Anspruch 1, wobei das Molverhältnis von Wasserstoff zu Kohlenmonoxid in dem Synthesegas vor dem Eintreten in den Oxoreaktor zwischen 1,01 und 1,05 liegt.

7. Verfahren nach Anspruch 1, wobei das Propylen zusammen mit dem Synthesegas in n-Butyraldehyd und iso-Butyraldehyd unter kontinuierlicher Hydroformylierungs-reaktion in dem Oxoreaktor umgewandelt wird.

8. Verfahren nach Anspruch 7, wobei das Propylen von außen und von dem nicht umgesetzten Propylen recycelt von den Stripp- und Stabilisierungseinheiten zugeführt wird.

9. Verfahren nach Anspruch 1, wobei aufgrund des kontinuierlichen Ersetzens von bis zu 15 Vol.-% von dem inaktiven Katalysator mit dem regenerierten Katalysator die Hydroformylierung als eine kontinuierliche Umsetzung durchgeführt wird.

10. Verfahren nach Anspruch 9, wobei das Katalysatorregenerierungssystem den inaktiven Katalysator mit dem regenerierten Katalysator ersetzt, wenn die Temperatur des Oxoreaktors 110°C übersteigt.

11. Verfahren nach Anspruch 1, wobei die Gase gelöst in dem Gemisch von Butyraldehyden einschließend Kohlenmonoxid, Wasserstoff, Methan, Propan und nicht umgesetztes Propylen in den Stripp- und Stabilisierungseinheiten nacheinander abgetrennt und dann in den Oxoreaktor rückgeführt werden.

12. Verfahren nach Anspruch 1, wobei das Gemisch von Aldehyden in die Trennungseinheit der Isomere eintritt, um iso-Butyraldehyd und n-Butyraldehyd voneinander zu trennen.

13. Verfahren nach Anspruch 1, wobei n-Butyraldehyd in 2-Ethyl-3-propylacrolein mit minimaler Umwandlung von 95% umgewandelt wird, nach Passieren durch das Aldolisierungssystem ausgestattet mit einem Kreislaufreaktor und einem Kreislaufwärmetauscher.

14. Verfahren nach Anspruch 1, wobei 2-Ethyl-3-propylacrolein in 2-Ethylhexanol mit minimaler Umwandlung von 94% in der Hydrierungseinheit, in welche der reine Wasserstoff von der Druckwechseladsorptionseinheit zugeführt wird, umgewandelt wird.

15. Verfahren nach Anspruch 1, wobei n-Butyraldehyd und iso-Butyraldehyd getrennt in n-Butanol beziehungsweise iso-Butanol mit einer Umwandlungsrate von 99% durch Hydrierungsreaktion, welcher der reine Wasserstoff von der Druckwechseladsorptionseinheit zugeführt wird, umgewandelt werden.

16. Verfahren nach Anspruch 1, wobei jeder der hergestellten Oxoalkohole einschließend 2-Ethylhexanol, n-Butanol und iso-Butanol kontinuierlich in der Trennungseinheit gereinigt wird.

17. Verfahren nach Anspruch 16, wobei Dampf, der für Wärmetauscher der Trennungseinheit erforderlich ist, durch die Dampferzeugungseinheit zugeführt wird.

18. Verfahren nach Anspruch 16, wobei die von der Trennungseinheit abgetrennten schwereren Alkohole als Brennstoff im Ofen der Reformierungseinheit verbraucht werden.

## Revendications

1. Procédé intégré de production d'oxo-alcools à partir de gaz naturel, comprenant les étapes suivantes :
a) conversion du gaz naturel désulfuré conjointement avec du dioxyde de carbone gazeux recyclé à partir de l'unité d'élimination de CO2 en gaz de synthèse dans l'unité de reformage ;
b) séparation du CO2 gazeux à partir du gaz de synthèse et son renvoi à l'unité de reformage ;
c) purification du gaz de synthèse afin de produire de l'hydrogène pur qui est utilisé pour l'hydrogénation des oxo-aldéhydes ;
d) production de n-butyraldéhyde et d'iso-butyraldéhyde par une réaction d'hydroformylation continue du propylène avec le gaz de synthèse dans le réacteur oxo ;
e) séparation des gaz dissous à partir du mélange de butyraldéhydes et leur renvoi au réacteur oxo ;
f) séparation de l'iso-butyraldéhyde et du n-butyraldéhyde l'un de l'autre ;
g) production de 2-éthyl-3-propylacroléine par aldolisation de n-butyraldéhyde ;
h) production de 2-éthylhexanol par hydrogénation de 2-éthyl-3-propylacroléine ;
i) production de n-butanol et d'iso-butanol par hydrogénation de n-butyraldéhyde et d'iso-butyraldéhyde ; et
j) purification continue des oxo-alcools produits comprenant le 2-éthylhexanol, le n-butanol et l'iso-butanol.

2. Procédé selon la revendication 1, dans lequel le gaz naturel est purifié dans l'unité de désulfuration en présence d'un catalyseur d'oxyde de molybdène/cobalt sur support d'alumine dans une plage de températures de 360 à 380°C jusqu'à une teneur maximale en soufre de 1 ppm, puis le gaz purifié, conjointement avec le dioxyde de carbone recyclé à partir de l'unité d'élimination de CO2, est converti en gaz de synthèse en présence de la vapeur et d'un catalyseur de nickel sur support d'alumine dans une plage de températures de 700 à 900°C et sous une pression de 12 à 16 bar dans l'unité de reformage.

3. Procédé selon la revendication 2, dans lequel la vapeur est fournie par l'unité de production de vapeur sous une pression de 15 à 25 bar et à une température de 200 à 250°C.

4. Procédé selon la revendication 1, dans lequel le dioxyde de carbone produit lors du processus de reformage est absorbé dans l'unité d'élimination de CO2 puis renvoyé à l'unité de reformage.

5. Procédé selon la revendication 1, dans lequel l'hydrogène pur est produit avec une pureté minimale de 99,5 % après passage du gaz de synthèse exempt de CO2 à travers l'unité d'adsorption par inversion de pression afin d'être utilisé dans l'hydrogénation des oxo-aldéhydes.

6. Procédé selon la revendication 1, dans lequel le rapport molaire entre l'hydrogène et le monoxyde de carbone dans le gaz de synthèse avant d'entrer dans le réacteur oxo est compris entre 1,01 et 1,05.

7. Procédé selon la revendication 1, dans lequel le propylène conjointement avec le gaz de synthèse est converti en n-butyraldéhyde et en iso-butyraldéhyde sous une réaction d'hydroformylation continue dans le réacteur oxo.

8. Procédé selon la revendication 7, dans lequel le propylène est fourni depuis l'extérieur et depuis le propylène non réagi recyclé à partir des unités de dégrippage et de stabilisation.

9. Procédé selon la revendication 1, dans lequel, en raison du remplacement continu de jusqu'à 15 % en volume du catalyseur inactif par le catalyseur régénéré, l'hydroformylation est effectuée sous la forme d'une réaction continue.

10. Procédé selon la revendication 9, dans lequel le système de régénération de catalyseur remplace le catalyseur inactif par le catalyseur régénéré lorsque la température du réacteur oxo dépasse 110°C.

11. Procédé selon la revendication 1, dans lequel les gaz dissous dans le mélange de butyraldéhydes comprenant le monoxyde de carbone, l'hydrogène, le méthane, le propane et le propylène non réagi se séparent successivement dans les unités de dégrippage et de stabilisation puis retournent au réacteur oxo.

12. Procédé selon la revendication 1, dans lequel le mélange d'aldéhydes entre dans l'unité de séparation des isomères afin de séparer l'iso-butyraldéhyde et le n-butyraldéhyde l'un de l'autre.

13. Procédé selon la revendication 1, dans lequel le n-butyraldéhyde est converti en 2-éthyl-3-propylacroléine avec une conversion minimale de 95 % après passage à travers le système d'aldolisation équipé d'un réacteur à boucle et d'un échangeur de chaleur à boucle.

14. Procédé selon la revendication 1, dans lequel la 2-éthyl-3-propylacroléine est convertie en 2-éthylhexanol avec une conversion minimale de 94 % dans l'unité d'hydrogénation dans laquelle l'hydrogène pur est fourni par l'unité d'adsorption par inversion de pression.

15. Procédé selon la revendication 1, dans lequel le n-butyraldéhyde et l'iso-butyraldéhyde sont convertis séparément en n-butanol et en iso-butanol, respectivement, avec un taux de conversion de 99 % par une réaction d'hydrogénation dans laquelle l'hydrogène pur est fourni par l'unité d'adsorption par inversion de pression.

16. Procédé selon la revendication 1, dans lequel chacun des oxo-alcools produits comprenant le 2-éthylhexanol, le n-butanol et l'iso-butanol est purifié en continu dans l'unité de séparation.

17. Procédé selon la revendication 16, dans lequel la vapeur requise pour les échangeurs de chaleur de l'unité de séparation est fournie par l'unité de production de vapeur.

18. Procédé selon la revendication 16, dans lequel les alcools plus lourds séparés de l'unité de séparation sont consommés comme combustible dans le four de l'unité de reformage.
